# EUROPEAN PATENT APPLICATION

(11) **EP 2 266 554 A1**
(43) Date of publication of application: **29.12.2010**
(21) Application number: 09290382.2
(22) Date of filing: 26.05.2009
(51) Int. Cl.: A61K 31/15, A61P 25/00

(54) **Method of treating sleep disorders using eplivanserin**

(71) Applicant: Sanofi-Aventis, 75013 Paris (FR)
(72) Inventor: Prieur, Amélie, 75013 Paris (FR); Rein, Werner, 75013 Paris (FR); Verpillat, Patrice, 75013 Paris (FR); Weinling, Estelle, 75013 Paris (FR)
(74) Representative: Morel-Pécheux, Muriel

(57) **Abstract**

Method for treating sleep disorders by using eplivanserin

Method of providing eplivanserin.

Method of managing the risk to allow an effective and safe use of eplivanserin Method of promoting the use of eplivanserin

Article of manufacture and package.

## Description

The instant invention relates to a method of treating sleep disorders by using eplivanserin or pharmaceutically acceptable salts or esters thereof in patients not displaying a prior history of diverticulitis.

The instant invention relates to a method of providing eplivanserin or pharmaceutically acceptable salts or esters thereof.

The instant invention also relates to a method of managing the risk of diverticulitis to allow an effective and safe use of eplivanserin or pharmaceutically acceptable salts or esters thereof of, in the treatment of patients treated for sleep disorders.

The instant invention also relates to a method of promoting the use of eplivanserin or pharmaceutically acceptable salts or esters thereof of.

The instant invention also relates to an article of manufacture and a package comprising eplivanserin or pharmaceutically acceptable salts or esters thereof.

It has been known for many years that neural pathways utilizing serotonin as their primary neurotransmitter play important roles in the control of sleep states. However, drugs which modulate serotoninergic transmission by, for example, stimulating or blocking receptors for this transmitter, have never found clinical use in the treatment of insomnia (Ann Pharm Fr 2007, vol. 65 pp. 268-274). This may soon change as several agents with selective activity as antagonists or inverse agonists at the 5HT_{2A} subtype of serotonin receptors are currently being tested in patients with sleep disorders. 5HT_{2A} antagonists do not show pharmacological effects similar to traditional sedative hypnotic agents. However, they do modify sleep architecture by selectively increasing slow wave sleep (Neuropsychopharmacology (1999); vol.21, pp. 455-466).

The compound (1Z, 2E)-1-(2-fluorophenyl)-3-(4-hydroxyphenyl)-prop-2-en-1-one-O-(2-dimethylaminoethyl)oxime, is hereafter referenced as eplivanserin or pharmaceutically acceptable salts or esters thereof. Documents EP 373998 and US 5166416 claim compounds having a generic scope encompassing eplivanserin or pharmaceutically acceptable salts or esters thereof and also specifically claimed eplivanserin or pharmaceutically acceptable salts or esters thereof, and disclosed its use as a platelet anti-aggregant or a psychotropic agent.

See also US 6143792 and EP 1014960 (sleep apnea) and US 6576970 and EP 11140955 (snoring and upper airway resistance syndrome).

Eplivanserin or pharmaceutically acceptable salts or esters thereof, in particular hemifumarate salt, is an antagonist of 5HT_{2A} receptors (Journal of Pharmacological Experiment in Therapeutics, (1992), vol. 262 (2), pp. 759-68). Eplivanserin is well absorbed (>70%). Conventional dosage, between 1 and 10 mg, leads to a maximal plasma concentration that is reached between 2 and 6 hours; the half-life time of eplivanserin or pharmaceutically acceptable salts or esters thereof is relatively long, with an average value of 50 hours. Eplivanserin or pharmaceutically acceptable salts or esters thereof is also known to enhance slow wave sleep (SWS) (Neuropsychopharmacology (1999), vol.21 (3), pp. 455-466).

It has been recently discovered that eplivanserin or pharmaceutically acceptable salts or esters thereof, by enhancing SWS, may significantly improve sleep maintenance and quality of sleep (QoS) without inducing next-day sedation and rebound or withdrawal symptoms after treatment discontinuation in patients with chronic insomnia and sleep maintenance difficulties.

Many people have small pouches in their colons that bulge outward through weak spots, like an inner tube that pokes through weak places in a tire. Each pouch is called a diverticulum. Pouches are called diverticula. The condition of having diverticula is called diverticulosis. The prevalence of diverticulosis is similar in men and women and increases with age, ranging from approximately 10 % in adults younger than 40 years of age to 50 to 70 % among those 80 years of age (N Engl J Med (2007) 357(20):2057-2066).
When the pouches become infected or inflamed, the condition is called diverticulitis. This happens in 10 to 25 percent of people with diverticulosis. Diverticulosis and diverticulitis are also called diverticular diseases.

Diverticular disease refers to symptomatic and asymptomatic disease with an underlying pathology of colonic diverticula. Approximatively 85 percent of patients with diverticula are believed to remain asymptomatic (Am. Fam. Physician (2005) 72:1229-1234, 1241-1242).

The most common symptom of diverticulitis is acute abdominal pain. The most common sign is tenderness around the left side of the lower abdomen. If infection is the cause, nausea, vomiting, fever, cramping, and constipation may occur as well. The severity of symptoms depends on the extent of the infection and complications. Diverticulitis worsens throughout the day, as it starts as small pains and slowly turns into vomiting and sharp pains (Am. Fam. Physician (2005) 72:1229-1234, 1241-1242).
Diagnosis is usually suggested by history and clinical exam and established with computerised tomography.

Acute diverticulitis can result in both immediate and long term complications. Complications include abscess formation, peritonitis, obstruction, fistula formation, and, rarely haemorrhage.

The severity of the inflammatory and infectious processes, as well as the underlying health of the patient, determines the appropriate treatment for patients with diverticulitis. Treatment for diverticulitis focuses on clearing up the infection and inflammation, resting the colon, and preventing or minimizing complications. An episode of diverticulitis without complications may respond to antibiotics within a few days if treated early. An acute episode with severe pain or severe infection may require a hospital stay. The antibiotics are given by injection into a vein. In some complication cases (abscess, fistula, bowel instruction and free perforation), however, surgery may be necessary.

After a first episode, about one third of patients will have a second episode of acute diverticulitis, and after a second episode another third will have yet another episode.

During clinical trials with eplivanserin in patients suffering from sleep disorders, and, in particular in patients suffering from insomnia characterized by difficulties with sleep maintenance, diverticulitis events have been reported. This occurred generally late after treatment initiation (two months on average).

Therefore the treatment of sleep disorders, in particular chronic insomnia characterized by sleep maintenance difficulties with eplivanserin or pharmaceutically acceptable salts or esters thereof is contra-indicated for patients displaying a prior history of diverticulitis.

The Applicant has found methods for managing the risk related to diverticulitis. The methods according to the invention enable to decrease the risk of a diverticulitis event, when eplivanserin or pharmaceutically acceptable salts or esters thereof is administered for treating sleep disorders.

One method according to the invention is performed by administering a therapeutic amount of eplivanserin or pharmaceutically acceptable salts or esters thereof in patients suffering from sleep disorders, and not displaying a prior history of diverticulitis. The invention thus concerns eplivanserin or pharmaceutically acceptable salts or esters thereof for the treatment of sleep disorders in patients suffering from sleep disorders, and not displaying a prior history of diverticulitis, a therapeutic amount of eplivanserin or pharmaceutically acceptable salts or esters thereof being administered. The invention thus concerns the use of eplivanserin or pharmaceutically acceptable salts or esters thereof for preparation of a medicament for the treatment of patients suffering from sleep disorders, and not displaying a prior history of diverticulitis. In some embodiments, a pharmaceutically acceptable salt of eplivanserin is hemifumarate.

In some embodiments, sleep disorders are insomnia.

In some embodiments, sleep disorders are chronic insomnia.

In some embodiments, sleep disorders are insomnia characterized by sleep maintenance difficulties.

In some embodiments, sleep disorders are insomnia including nocturnal awakenings, usually for short-term duration.

In some embodiments, sleep disorders are insomnia including nocturnal awakenings or early awakenings, usually for short-term duration. In some embodiments, sleep disorders are chronic insomnia characterized by difficulties with sleep maintenance.

Another method according to the invention is performed by providing eplivanserin or pharmaceutically acceptable salts or esters thereof, wherein said eplivanserin or pharmaceutically acceptable salts or esters thereof is provided along with information indicating that eplivanserin is useful for treating patients suffering from sleep disorders and not displaying a prior history of diverticulitis.

In some embodiments, the information comprises printed matter that advises that eplivanserin or pharmaceutically acceptable salts or esters thereof is useful for treating patients suffering from sleep disorders and not displaying a prior history of diverticulitis. In some embodiments, said printed matter is a label.

The instant invention also relates to a method of managing the risk of diverticulitis to allow an effective and safe use of eplivanserin or pharmaceutically acceptable salts or esters thereof in the treatment of patients treated for sleep disorders, comprises the following steps:
a) Initially check the patients suffering from sleep disorders,
b) if abdominal pain associated with altered gastrointestinal mobility or fever is detected during the treatment with eplivanserin or pharmaceutically acceptable salts or esters thereof, then the treatment should be discontinued;
c) if, after a diagnosis of diverticulitis is excluded, treatment with eplivanserin or pharmaceutically acceptable salts or esters thereof should be reinitiated, patients should be closely monitored.

In some embodiments of the above method, abdominal pain is in the lower left quadrant, associated with altered gastrointestinal mobility such as constipation and/or diarrhea, or fever.

Another method according to the invention consists in promoting the use of eplivanserin or pharmaceutically acceptable salts or esters thereof, said method comprising the step of conveying to a recipient at least one message selected from the group consisting of:
(a) eplivanserin or pharmaceutically acceptable salts or esters thereof should be prescribed to a patient who has not been diagnosed with diverticulitis;
(b) eplivanserin or pharmaceutically acceptable salts or esters thereof should be prescribed to a patient who does not have a prior history of diverticulitis;
(c) eplivanserin or pharmaceutically acceptable salts or esters thereof is contraindicated in patients with a prior history of diverticulitis;
(d) if abdominal pain associated with altered gastrointestinal mobility or fever is detected during the treatment with eplivanserin or pharmaceutically acceptable salts, then the treatment with eplivanserin or pharmaceutically acceptable salts or esters thereof should be discontinued.

The invention also relates to an article of manufacture comprising
a) a packaging material;
b) eplivanserin or pharmaceutically acceptable salts or esters thereof or, and
c) a label or package insert contained within the packaging material indicating that:
   i) eplivanserin or pharmaceutically acceptable salts or esters thereof is contraindicated in patients with a history of diverticulitis and
   ii) if abdominal pain associated with altered gastrointestinal mobility or fever is detected during the treatment with eplivanserin or pharmaceutically acceptable salts or esters thereof, then the treatment with eplivanserin or pharmaceutically acceptable salts or esters thereof should be discontinued.

In some embodiments, a pharmaceutically acceptable salt of eplivanserin is hemifumarate.

The invention also relates to a package comprising eplivanserin or pharmaceutically acceptable salts or esters thereof and a label, said label comprising a printed statement which informs a prospective user that:
i) eplivanserin or pharmaceutically acceptable salts or esters thereof is contraindicated in patients with a history of diverticulitis and
ii) if abdominal pain associated with altered gastrointestinal mobility or fever is detected during the treatment with eplivanserin or pharmaceutically acceptable salts or esters thereof, then the treatment with eplivanserin or pharmaceutically acceptable salts or esters thereof should be discontinued.

In some embodiments, a pharmaceutically acceptable salt of eplivanserin is hemifumarate.

In some embodiments of all the methods according to the invention, a pharmaceutically acceptable salt of eplivanserin is hemifumarate.

In some embodiments of all the methods according to the invention, sleep disorders are insomnia.

In some embodiments of all the methods according to the invention, sleep disorders are chronic insomnia.

In some embodiments of all the methods according to the invention, sleep disorders are insomnia characterized by sleep maintenance difficulties.

In some embodiments of all the methods according to the invention, sleep disorders are insomnia including nocturnal awakenings, usually for short-term duration.

In some embodiments of all the methods according to the invention, sleep disorders are insomnia including nocturnal awakenings or early awakenings, usually for short-term duration. In some embodiments of all the methods according to the invention, sleep disorders are chronic insomnia characterized by difficulties with sleep maintenance.

The term "treating" or "treat" refers to either preventing, providing symptomatic relief, or curing the patient's disease, disorder or condition.

In the cbntext of the present patent application, the term "sleep disorders" especially means, insomnia, primary insomnia, sleep maintenance insomnia, insomnia associated with a mental disease, comorbid insomnia, i.e. insomnia associated with sleep apnea, pain, diabetes, depression, anxiety.

The term "insomnia" (Diagnostic and Statistical Manual of Mental Disorders, fourth edition criteria) includes difficulty in maintaining sleep, with multiple nocturnal awakenings.

The term "administering" comprises administration via any appropriate unitary dosage forms for eplivanserin or pharmaceutically acceptable salts or esters thereof such as oral forms, such as tablets, hard or soft gelatin capsules, powders, granules and oral solutions or suspensions, as well as the sublingual, buccal, intratracheal, intraocular, intranasal forms, the forms adapted to inhalation, topical, transdermal, sub-cutaneous, intramuscular or intravenous delivery, the rectal forms and the implants. For the topical application, eplivanserin or pharmaceutically acceptable salts or esters thereof may be used as creams, gels, ointments or lotions.

For eplivanserin or pharmaceutically acceptable salts or esters thereof, tablets can be a preferred mode of administration.

The term "therapeutic amount" means enough of the compound which becomes available through the appropriate route of administration to treat the patient for the disorder, the condition or the disease.

For eplivanserin or pharmaceutically acceptable salts or esters thereof, a therapeutic amount is 5 mg once a day. In some embodiments, the administration can be done either in the morning or in the evening, just before a sleep period, or at any time of the day.

However in specific cases, different dosages may be appropriate; these dosages are comprised within the scope of the present invention. According to usual practice, the dosage suitable to each patient is determined by the physician according to, for example, the administration route, the weight and response of the patient.

By "discontinued", one may mean temporary interruption of the treatment for one period (until diagnosis of diverticulitis is confirmed or excluded) or complete interruption of the treatment.

The term "providing" includes selling, distributing, shipping, offering for sell, importing etc.

The history of diverticulosis and/or diverticulitis may be established using a routine patient medical questionnaire.

The instant invention is illustrated by the clinical data below.

### Example 1:

The effects of eplivanserin or pharmaceutically acceptable salts or esters thereof on sleep architecture and sleep parameters in patients with sleep disorders were examined.

The Phase 3 program of development of eplivanserin in the treatment of patients with chronic (primary) insomnia characterized by sleep maintenance difficulties consisted of 3 randomized, double-blind (DB), placebo-controlled, efficacy and safety studies designed to assess the efficacy of eplivanserin on sleep maintenance either using polysomnography (PSG) recordings (6-week study EFC6220) or patient-reported outcome (12-week studies LTE6217 and LTE6262) collected daily on sleep questionnaires.

They were all double-blind (DB), randomized, placebo-controlled studies. In all studies, treatment period was preceded by a one week, placebo run-in period, designed to check selection criteria for insomnia based on patient reported sleep parameters collected on the daily sleep questionnaires (Studies LTE6217, and LTE6262) or based on PSG recordings during two screening nights in a sleep laboratory (Study EFC6220). Treatment period was followed by a placebo run-out period of 2 weeks designed to assess the effect of abrupt treatment discontinuation.

Study EFC6220 was designed to measure the pharmacodynamic activity of eplivanserin on sleep maintenance using PSG recordings in a sleep laboratory. Polysomnograms were recorded in standard conditions during 8 hours in bed.

Studies LTE6217 and LTE6262 measured the efficacy of eplivanserin in real life conditions of use at home by collecting daily sleep parameters reported by patients through an Interactive Voice Response System (IVRS).

The results of Study EFC6220 confirmed the expected clinical activity of eplivanserin on sleep maintenance by decreasing PSG-WASO (Wake after sleep onset) at Week 3 and Week 6, but the difference with placebo did not reach statistical significance at Week 6. However, eplivanserin 5 mg was shown to consistently decrease PSG-NAW (number of awakenings) at both time points (LS mean =-1.74 and -1.82, p<0.0001 at Week 3 and Week 6 respectively), associated with a decrease of pr-NAW (patient-reported number of awakenings). Eplivanserin decreased the PSG-NAW mainly from H1 up to H6 compared with placebo. The decrease of wakefulness was not associated with an increase of the final awakening. The duration of the remaining awakenings was not increased. In addition, eplivanserin decreased the number of brief awakenings (<1 min) as compared to placebo confirming its effect on sleep consolidation.

In addition, the QoS (quality of sleep) was improved and a majority of patients declared that eplivanserin "helped them sleep" (PGI-item 1) and "increased their duration of sleep" (PGI-item 3). Comparable results were observed in the elderly patients (≥65 years).

Eplivanserin 5 mg did not affect sleep onset as shown by the results of PSG-LPS and pr-SOL (sleep onset latency).

The analysis of sleep architecture showed an increased percentage of time spent in sleep Stages 3&4 (slow wave sleep or deep sleep) with eplivanserin 5 mg/day compared with placebo, associated mainly with a decrease of Stage 1 (a stage close to wakefulness). The per hour analysis of SWS showed that eplivanserin increased SWS from H2 to H6 in comparison with placebo with a maximum at H3 while respecting the biological structure of sleep. The increase of SWS/WASO + Stage 1 index observed with eplivanserin also confirmed the decrease of sleep fragmentation and improved quality of sleep with eplivanserin. REM (rapid eye movement) sleep was not affected by eplivanserin.

As shown through Tables 1 and 2, Eplivanserin 5 mg/day improved sleep maintenance by consistently and significantly decreasing pr-WASO (studies LTE6217 and LTE6262), reducing the mean pr-NAW and increasing the pr-TST (total sleep time) after 6 and 12 weeks of treatment, as compared to placebo. Eplivanserin also improved the Quality of Sleep as well as the Refreshing Quality of Sleep after 6 and 12 weeks of treatment, in patients with insomnia characterized by sleep maintenance difficulties. Eplivanserin did not affect sleep onset.

Tables 1 and 2 show study results for the 12-week studies LTE6217 and LTE6262.

In these tables, values are given as an amount of time expressed in minutes and seconds (mm:ss). As an example, in Table 2, for pr-WASO at 12 weeks, the value "-53:17" displays a mean decrease of patient-reported wake time after sleep onset of 53 minutes and 17 seconds.

**Table 1 - pr-WASO (min:sec), pr-TST (min:sec), pr-NAW, pr-SOL (min:sec), pr-QoS, pr-refreshing quality of sleep at Week 6 and 12 of treatment using MMRM analysis - ITT population - Study LTE6217**

| | | | | **Difference from Placebo** | | |
|---|---|---|---|---|---|---|
| | | **Placebo** **(N=345)** **LS Mean** **(SEM)** | **Epli 5 mg/day** **(N=617)** **LS Mean** **(SEM)** | **LS Mean** | **95%** **Confidence Interval** | **p-value** |
| pr-WASO | Week 6 | -35:44 (2:08) | -48:50 (1:35) | -13:06 | (-18:19 to -7:53) | <.0001 |
| | Week 12 | -42:48 (2:15) | -54:20 (1:40) | -11:32 | (-17:03 to -6:02) | <.0001 |
| pr-TST | Week 6 | 33:10 (2:52) | 44:15 (2:08) | 11:05 | (4:03 to 18:06) | 0.0020 |
| | Week 12 | 38:28 (3:12) | 49:24 (2:22) | 10:56 | (3:06 to 18:45) | 0.0062 |
| pr-NAW | Week 6 | -0.77 (0.05) | -1.16 (0.04) | -0.39 | (-0.52 to -0.26) | <.0001 |
| | Week 12 | -0.93 (0.06) | -1.27 (0.04) | -0.33 | (-0.48 to -0.19) | <.0001 |
| pr-SOL | Week 6 | 0:18 (0:34) | -0:23 (0:25) | -0:41 | (-2:04 to 0:42) | 0.3299 |
| | Week 12 | 0:04 (0:44) | -0:35 (0:32) | -0:39 | (-2:25 to 1:07) | 0.4729 |
| Quality of sleep | Week 6 | -0.54 (0.03) | -0.65 (0.02) | -0.11 | (-0.18 to -0.03) | 0.0054 |
| | Week 12 | -0.62 (0.03) | -0.73 (0.03) | -0.10 | (-0.19 to -0.02) | 0.0146 |
| Refreshing quality of sleep | Week 6 | -0.51 (0.03) | -0.62 (0.02) | -0.11 | (-0.18 to -0.03) | 0.0046 |
| | Week 12 | -0.60 (0.03) | -0.70 (0.03) | -0.09 | (-0.18to -0.01) | 0.0303 |

| | | | | | | |
|---|---|---|---|---|---|---|
| Note: PR: patient reported, TST: total sleep time, NAW: number of awakening, SOL: sleep onset latency N is the number of patients in the ITT population p-values come from MMRM analysis adjusting for baseline value | | | | | | |

**Table 2 - pr-WASO (min:sec), pr-TST (min:sec), pr-NAW, pr-SOL (min:sec), pr-QoS, pr-refreshing quality of sleep at Week 6 and 12 of treatment using MMRM analysis - ITT population - Study LTE6262**

| | | | | **Difference from Placebo** | | |
|---|---|---|---|---|---|---|
| | | **Placebo** **(N=295)** | **Epli 5 mg/day** **(N=850)** | **LS Mean** | **95% Confidence Interval** | **p-value** |
| | | **LS Mean (SEM)** | **LS Mean (SEM)** | | | |
| pr-WASO | Week 6 | -33:34 (2:30) | -47:55 (1:28) | -14:21 | (-20:01 to -8:40) | <.0001 |
| | Week 12 | -39:46 (2:33) | -53:17 (1:30) | -13:31 | (-19:19 to -7:43) | <.0001 |
| pr-TST | Week 6 | 29:13 (2:50) | 42:34 (1:40) | 13:20 | (6:52 to 19:49) | <.0001 |
| | Week 12 | 30:50 (3:07) | 47:10 (1:51) | 16:20 | (9:14 to 23:27) | <.0001 |
| pr-NAW | Week 6 | -0.66 (0.08) | -0.99 (0.05) | -0.34 | (-0.52 to -0.16) | 0.0002 |
| | Week 12 | -0.74 (0.07) | -1.08 (0.04) | -0.35 | (-0.50 to -0.19) | <.0001 |
| pr-SOL | Week 6 | 1:46 (0:42) | 0:23 (0:25) | -1:23 | (-2:58 to 0:12) | 0.0860 |
| | Week 12 | 1:50 (0:45) | 0:08 (0:27) | -1:42 | (-3:25 to 0:02) | 0.0542 |
| Quality of sleep | Week 6 | -0.46 (0.03) | -0.58 (0.02) | -0.13 | (-0.20 to -0.05) | 0.0006 |
| | Week 12 | -0.49 (0.03) | -0.63 (0.02) | -0.14 | (-0.21 to -0.06) | 0.0004 |
| Refreshing QoS | Week 6 | -0.41 (0.03) | -0.55 (0.02) | -0.13 | (-0.20 to -0.06) | 0.0004 |
| | Week 12 | -0.45 (0.03) | -0.60 (0.02) | -0.15 | (-0.23 to -0.07) | 0.0002 |

| | | | | | | |
|---|---|---|---|---|---|---|
| Note: PR: patient reported, TST: total sleep time, NAW: number of awakening, SOL: sleep onset latency N is the number of patients in the ITT population p-values come from MMRM analysis adjusting for baseline value | | | | | | |

Patients reported that they perceived eplivanserin 5 mg/day as an "aid to sleep" (PGI-item 1) and as "increasing duration of sleep" (PGI-item 3) in comparison with placebo, after 6 and 12 weeks of treatment. In addition, a positive effect of eplivanserin compared with placebo was observed regarding the improvement of activities related to work and hobby activities after 12 weeks of treatment in Study LTE6262 and the meta-analysis of the 2 long-term studies (LTE6262 and LTE6217).

Results from the safety analyses on potential for residual effects not only showed that eplivanserin was devoid of next-day impairment but that eplivanserin was reported by insomniac patients to have a significantly favourable effect on "sleepiness in the morning" and "ability to concentrate" as compared with placebo.

Results in the elderly sub-population were consistent with those of the global population.

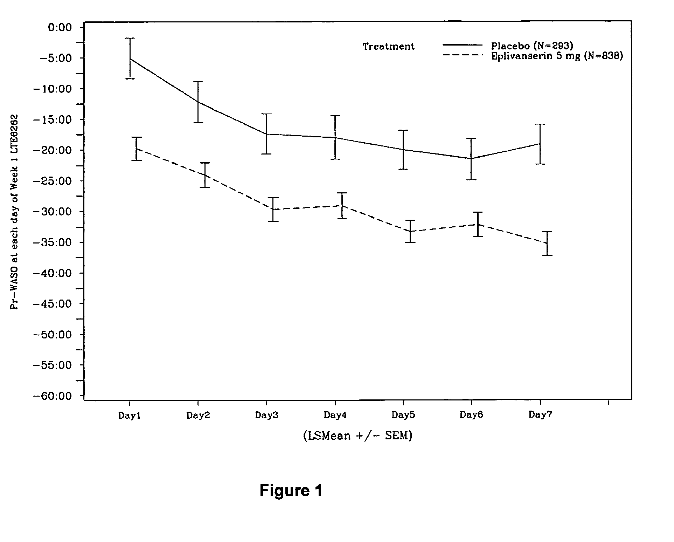

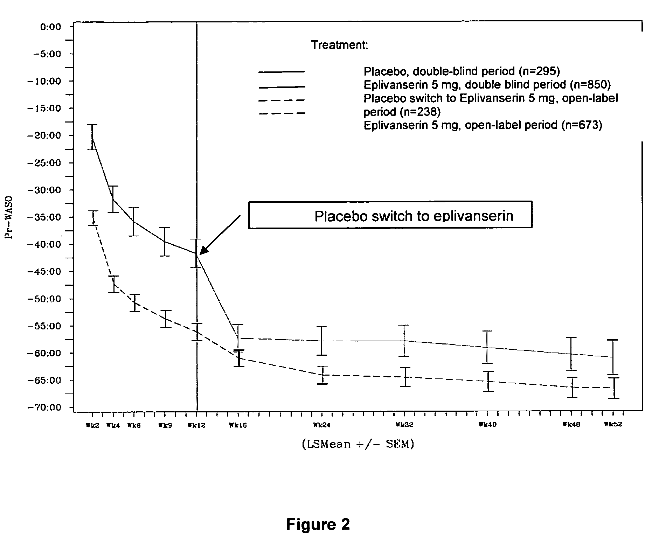

The effect of eplivanserin 5 mg/day on sleep maintenance was observed from Day 1 and persisted up to 1 year of treatment with a stable decreased of mean change from baseline for pr-WASO and no apparent dose increase. These results indicate that eplivanserin has an early onset of action and a maintained efficacy potential for the long-term treatment of chronic insomnia. (Figure 1 and 2 Table 3 and 4) Figures 1 and 2 show the effect of eplivanserin 5 mg/day on sleep maintenance during the first week of treatment (figure 1) and during 52 weeks (figure 2).

### Figure 1:

pr-WASO mean changes from baseline ± standard error of mean during the first week of treatment (MMRM estimates) - Study LTE6262

### Figure 2:

pr-WASO mean change from baseline ± standard error of mean by visit during the double-blind and open-label periods (MMRM estimates) - Study LTE6262

**Table 3 - Study LTE6262: pr-WASO (min:sec): Change from baseline per day during Week 1 (MMRM analysis) - ITT population**

| | **Placebo** **(N=295)** | **Eplivanserin** **5mg/day** **(N=850)** |
|---|---|---|
| Number | 293 | 838 |
| | | |
| Baseline | | |
| Mean (SD) | 108:32 (41:10) | 112:08 (44:53) |
| Median | 100:00 | 102:01 |
| Min : Max | 46:10: 246:00 | 41:40: 315:00 |
| | | |
| Change from baseline at day 1 | | |
| LS Mean (SEM) | -5:05 (3:17) | -19:47 (1:55) |
| LS Mean Difference from placebo (SEM) | | -14:42 (3:48) |
| 95% Confidence Interval | | (-22:10 to -7:14) |
| p-value vs. Placebo | | 0.0001 |
| | | |
| Change from baseline at day 2 | | |
| LS Mean (SEM) | -12:12 (3:22) | -24:06 (2:00) |
| LS Mean Difference from placebo (SEM) | | -11:54 (3:55) |
| 95% Confidence Interval | | (-19:36 to -4:12) |
| p-value vs. Placebo | | 0.0025 |
| | | |
| Change from baseline at day 3 | | |
| LS Mean (SEM) | -17:27 (3:17) | -29:43 (1:56) |
| LS Mean Difference from placebo (SEM) | | -12:17 (3:49) |
| 95% Confidence Interval | | (-19:46 to -4:48) |
| p-value vs. Placebo | | 0.0013 |
| | | |
| Change from baseline at day 4 | | |
| LS Mean (SEM) | -18:02 (3:32) | -29:09 (2:04) |
| LS Mean Difference from placebo (SEM) | | -11:07 (4:05) |
| 95% Confidence Interval | | (-19:08 to -3:05) |
| p-value vs. Placebo | | 0.0067 |
| | | |
| Change from baseline at day 5 | | |
| LS Mean (SEM) | -20:01 (3:10) | -33:20 (1:51) |
| LS Mean Difference from placebo (SEM) | | -13:18 (3:40) |
| 95% Confidence Interval | | (-20:31 to -6:06) |
| p-value vs. Placebo | | 0.0003 |
| | | |
| Change from baseline at day 6 | | |
| LS Mean (SEM) | -21:35 (3:22) | -32:15 (1:59) |
| LS Mean Difference from placebo (SEM) | | -10:39 (3:54) |
| 95% Confidence Interval | | (-18:19 to -2:59) |
| p-value vs. Placebo | | 0.0065 |
| | | |
| Change from baseline at day 7 | | |
| LS Mean (SEM) | -19:09 (3:17) | -35:20 (1:55) |
| LS Mean Difference from placebo (SEM) | | -16:11 (3:48) |
| 95% Confidence Interval | | (-23:39 to -8:43) |
| p-value vs. Placebo | | <.0001 |

| | | |
|---|---|---|
| Note: pr-WASO: patient reported wake time after sleep onset Number is the number of patients for the given parameter with no missing data, baseline value is used as a covariate in MMRM analysis p-values come from MMRM analysis adjusting for baseline value | | |

**Table 4 - Study LTE6262: pr-WASO (min:sec): Change from baseline per week during 12 weeks (MMRM analysis) - ITT population**

| | **Placebo** **(N=295)** | **Eplivanserin** **5mg/day** **(N=850)** |
|---|---|---|
| Number | 293 | 838 |
| | | |
| Baseline | | |
| Mean (SD) | 108:32 (41:10) | 112:08 (44:53) |
| Median | 100:00 | 102:01 |
| Min : Max | 46:10: 246:00 | 41:40: 315:00 |
| | | |
| Change from baseline at week 1 | | |
| LS Mean (SEM) | -15:56 (2:06) | -29:00 (1:15) |
| LS Mean Difference from placebo (SEM) | | -13:04 (2:27) |
| 95% Confidence Interval | | (-17:52 to -8:16) |
| p-value vs. Placebo | | <.0001 |
| | | |
| Change from baseline at week 2 | | |
| LS Mean (SEM) | -23:34 (2:22) | -38:41 (1:24) |
| LS Mean Difference from placebo (SEM) | | -15:07 (2:45) |
| 95% Confidence Interval | | (-20:31 to -9:43) |
| p-value vs. Placebo | | <.0001 |
| | | |
| Change from baseline at week 3 | | |
| LS Mean (SEM) | -29:11 (2:21) | -43:03 (1:24) |
| LS Mean Difference from placebo (SEM) | | -13:53 (2:44) |
| 95% Confidence Interval | | (-19:15 to -8:31) |
| p-value vs. Placebo | | <.0001 |
| | | |
| Change from baseline at week 4 | | |
| LS Mean (SEM) | -29:40 (2:26) | -46:57 (1:26) |
| LS Mean Difference from placebo (SEM) | | -17:17 (2:50) |
| 95% Confidence Interval | | (-22:50 to -11:44) |
| p-value vs. Placebo | | <.0001 |
| | | |
| Change from baseline at week 5 | | |
| LS Mean (SEM) | -33:56 (2:36) | -48:09 (1:32) |
| LS Mean Difference from placebo (SEM) | | -14:13 (3:01) |
| 95% Confidence Interval | | (-20:09 to -8:17) |
| p-value vs. Placebo | | <.0001 |
| | | |
| Change from baseline at week 6 | | |
| LS Mean (SEM) | -31:54 (2:40) | -48:36 (1:34) |
| LS Mean Difference from placebo (SEM) | | -16:42 (3:05) |
| 95% Confidence Interval | | (-22:45 to -10:38) |
| p-value vs. Placebo | | <.0001 |
| | | |
| Change from baseline at week 7 | | |
| LS Mean (SEM) | -37:53 (2:37) | -49:59 (1:33) |
| LS Mean Difference from placebo (SEM) | | -12:06 (3:02) |
| 95% Confidence Interval | | (-18:03 to -6:08) |
| p-value vs. Placebo | | <.0001 |
| | | |
| Change from baseline at week 8 | | |
| LS Mean (SEM) | -36:43 (2:38) | -51:39 (1:34) |
| LS Mean Difference from placebo (SEM) | | -14:56 (3:04) |
| 95% Confidence Interval | | (-20:57 to -8:55) |
| p-value vs. Placebo | | <.0001 |
| | | |
| Change from baseline at week 9 | | |
| LS Mean (SEM) | -35:55 (2:44) | -51:46 (1:37) |
| LS Mean Difference from placebo (SEM) | | -15:52 (3:11) |
| 95% Confidence Interval | | (-22:06 to -9:37) |
| p-value vs. Placebo | | <.0001 |
| | | |
| Change from baseline at week 10 | | |
| LS Mean (SEM) | -39:34 (2:40) | -53:39 (1:35) |
| LS Mean Difference from placebo (SEM) | | -14:06 (3:06) |
| 95% Confidence Interval | | (-20:11 to -8:00) |
| p-value vs. Placebo | | <.0001 |
| | | |
| Change from baseline at week 11 | | |
| LS Mean (SEM) | -39:38 (2:43) | -53:44 (1:36) |
| LS Mean Difference from placebo (SEM) | | -14:06 (3:09) |
| 95% Confidence Interval | | (-20:17 to -7:55) |
| p-value vs. Placebo | | <.0001 |
| | | |
| Change from baseline at week 12 | | |
| LS Mean (SEM) | -38:44 (2:43) | -53:40 (1:37) |
| LS Mean Difference from placebo (SEM) | | -14:56 (3:09) |
| 95% Confidence Interval | | (-21:08 to -8:44) |
| p-value vs. Placebo | | <.0001 |
| Change from baseline at week 5 | | |
| LS Mean (SEM) | -33:56 (2:36) | -48:09 (1:32) |
| LS Mean Difference from placebo (SEM) | | -14:13 (3:01) |
| 95% Confidence Interval | | (-20:09 to -8:17) |
| p-value vs. Placebo | | <.0001 |
| | | |
| Change from baseline at week 6 | | |
| LS Mean (SEM) | -31:54 (2:40) | -48:36 (1:34) |
| LS Mean Difference from placebo (SEM) | | -16:42 (3:05) |
| 95% Confidence Interval | | (-22:45 to -10:38) |
| p-value vs. Placebo | | <.0001 |
| | | |
| Change from baseline at week 7 | | |
| LS Mean (SEM) | -37:53 (2:37) | -49:59 (1:33) |
| LS Mean Difference from placebo (SEM) | | -12:06 (3:02) |
| 95% Confidence Interval | | (-18:03 to -6:08) |
| p-value vs. Placebo | | <.0001 |
| | | |
| Change from baseline at week 8 | | |
| LS Mean (SEM) | -36:43 (2:38) | -51:39 (1:34) |
| LS Mean Difference from placebo (SEM) | | -14:56 (3:04) |
| 95% Confidence Interval | | (-20:57 to -8:55) |
| p-value vs. Placebo | | <.0001 |
| | | |
| Change from baseline at week 9 | | |
| LS Mean (SEM) | -35:55 (2:44) | -51:46 (1:37) |
| LS Mean Difference from placebo (SEM) | | -15:52 (3:11) |
| 95% Confidence Interval | | (-22:06 to -9:37) |
| p-value vs. Placebo | | <.0001 |
| | | |
| Change from baseline at week 10 | | |
| LS Mean (SEM) | -39:34 (2:40) | -53:39 (1:35) |
| LS Mean Difference from placebo (SEM) | | -14:06 (3:06) |
| 95% Confidence Interval | | (-20:11 to -8:00) |
| p-value vs. Placebo | | <.0001 |
| | | |
| Change from baseline at week 11 | | |
| LS Mean (SEM) | -39:38 (2:43) | -53:44 (1:36) |
| LS Mean Difference from placebo (SEM) | | -14:06 (3:09) |
| 95% Confidence Interval | | (-20:17 to -7:55) |
| p-value vs. Placebo | | <.0001 |
| | | |
| Change from baseline at week 12 | | |
| LS Mean (SEM) | -38:44 (2:43) | -53:40 (1:37) |
| LS Mean Difference from placebo (SEM) | | -14:56 (3:09) |
| 95% Confidence Interval | | (-21:08 to -8:44) |
| p-value vs. Placebo | | <.0001 |

| | | |
|---|---|---|
| Note: pr-WASO: patient reported wake time after sleep onset Number is the number of patients for the given parameter with no missing data, baseline value is used as a covariate in MMRM analysis p-values come from MMRM analysis adjusting for baseline value | | |

These results showed that eplivanserin promoted sleep maintenance by enhancing the deep component of sleep (SWS) and by decreasing the "hyper-arousal" state of insomniac patients and sleep fragmentation offering patients a more consolidated sleep. The analysis of sleep architecture also allowed the demonstration that, unlike benzodiazepine hypnotics, eplivanserin preserved and even restored the physiological structure of sleep in insomniac patients. Finally, patients perceived the decrease in the WASO and NAW and reported that eplivanserin improved their sleep quality and refreshing sleep.

### Example 2:

The effects of eplivanserin or pharmaceutically acceptable salts or esters thereof on sleep and daytime functioning in healthy subjects were examined.

Double-blind, randomized, placebo (PBO)-controlled, 4-period, cross-over study with a 1-2-week washout between each period. Single oral doses of eplivanserin or pharmaceutically acceptable salts or esters thereof 1, 10 and 40 mg and PBO were given to healthy male subjects in either the evening (pm) or the morning (am; n=16). Sleep was assessed objectively using an EEG and subjectively by Leeds Sleep Evaluation Questionnaire. Next-day psychomotor function, memory and arousal were measured objectively via Critical Flicker Fusion (CFF), Choice Reaction Time, Compensatory Tracking and Sternberg memory-scanning tasks. Subjective assessment of drug activity was performed using Line Analogue Rating Scales.

Irrespective of eplivanserin or pharmaceutically acceptable salts or esters thereof dose, both morning and evening administration significantly increased 'deep'/slow-wave sleep (SWS) time and decreased Stage 2 (light) sleep (*P*=0.0001 vs PBO for each). Sleep efficiency (time asleep/time in bed) was also significantly improved by EPL (*P*=0.0004 vs PBO), and the number of awakenings ≥120 s was significantly reduced (*P*=0.03 vs PBO). Times to sleep onset and total sleep time were unaffected in these healthy subjects. There were no detrimental effects on next-day objective psychomotor function or memory tests, or subjective ratings of 'hangover' or central nervous system (CNS) sedation, regardless of dose, or am or pm administration. A slight decrease in CFF threshold detection was observed at all doses, likely related to a direct effect on pupillary response (seen with other 5HT₂ antagonists). No serious or severe treatment-emergent AEs were reported. Regardless of dosage or dose timing (morning or evening), eplivanserin or pharmaceutically acceptable salts or esters thereof doubled SWS in healthy subjects without impairing daytime psychomotor function or short-term memory or inducing subjective ratings of CNS impairment and hangover.

### Example 3:

Hereafter are the observations made in pooled phase 2 and 3 trials with eplivanserin or pharmaceutically acceptable salts or esters thereof.

A total of 30 patients (30/3030, 1 %) exposed to eplivanserin have developed diverticulitis (all Phase 2-3 studies, eplivanserin all doses), corresponding to an exposure adjusted incidence rate of 3.32 patients with an event per 100 patient-years. No cases of diverticulitis were reported in the placebo-treated population. The cumulative incidence rate of diverticulitis (all eplivanserin population, all doses) increased over time up to an incidence rate of about 1.6% at Week 24. None of the more than 500 patients still on eplivanserin after 24 weeks developed diverticulitis after that time.

The demographic and baseline characteristics (age, race, body weight, creatinine clearance status at baseline) of the eplivanserin population (Pool 2) were comparable with those of the placebo population, except for a medical history of diverticular disease and concomitant treatment with opiate analgesics more frequently reported in eplivanserin patients, and IBS and functional colopathy more frequently reported in placebo patients. The majority of patients were non-elderly, females, and Caucasian with a median age between 47 and 51 years across treatment groups.

The demographic and baseline characteristics of the overall eplivanserin-treated patients (3030 patients) were comparable to those observed in the eplivanserin-treated population (Pool 2).

The demographic and baseline characteristics of the 30 eplivanserin-treated patients who developed diverticulitis were comparable to those observed in the overall eplivanserin-treated population, except for a higher mean/median age (median: 62 years versus 50 years). There were 18 female and 12 male patients reflecting the gender ratio of the overall population.

Subgroup analyses, confirmed a higher incidence rate of diverticulitis in elderly patients (≥65 years), in patients with a medical history of diverticulitis or diverticular disease and in patients with concomitant opioids intake (see table 1).

The daily dose of eplivanserin was of 5 mg in 29 patients and 0.2 mg in 1 patient. There were 7 serious and 23 non-serious cases. The 7 cases were assessed as serious based on the "involved or prolonged inpatient hospitalization" ICH seriousness criterion. The reported reason for hospitalization was abdominal pain in 6 patients and 1 patient was hospitalized for surgery as part of the management of the diverticulitis.

A total of 39 episodes of diverticulitis were reported in the 30 patients. The number of episodes of diverticulitis, per patient, varied from 1 to 4. The majority of the patients had a single episode of diverticulitis (23/30 patients). Six patients had 2 episodes and 1 patient had 4 episodes. Five out of the 7 patients who experienced recurrent diverticulitis had a medical history of diverticulitis/diverticular disease. In the 7 patients with recurrent episodes, the period of time between episodes varied from 7 days to 6 months. Three out of 39 episodes of diverticulitis were of mild intensity, 26/39 were moderate and 10/39 were severe, as per Investigator's judgment. The majority of cases occurred between 6 and 12 weeks of eplivanserin exposure (median time to onset: 58 days). The duration of diverticulitis episodes (from the onset of the event) varied from 24-48 hours (in 1 patient) to 116 days (median: 17 days, mean: 23 days).

The diagnosis of diverticulitis was made based on clinical symptoms with positive complementary radiological tests (abdominal ultrasound, colonoscopy, barium enema, CT scan) in 20/30 patients and on clinical symptoms only in 10/30 patients. Abdominal pain was the most frequently reported symptom (25/30). Changes in bowel motility were reported in 14 patients i.e., constipation in 8 patients and diarrhoea in 6 patients.

Fifteen out of 30 patients took concomitant medications thought to increase the risk of diverticulitis or episodes (NSAIDs, aspirin, corticosteroids, and opioids) (see table 1). Corrective treatment with antibiotic therapy was reported in 27/30 patients. One of them had additional surgery (anterior resection of sigmoid colon) as corrective treatment for the management of recurrent episodes of diverticulitis (4 episodes during the study) that did not resolve after repeated courses of antibiotic therapy. In the remaining 3 patients, no antibiotic corrective treatment was reported (the outcome of diverticulitis episode in these 3 patients was reported as recovered).

Recovery was reported as final outcome in all patients, except 2 (who had not recovered at the end of the study visit). None led to treatment discontinuation. Four patients recovered while the study drug had been stopped or discontinued for another reason and in 4 patients the treatment was discontinued prior (24 to 48 hours) to the onset of the episode.

### Conclusions:

The analysis of cases of diverticulitis showed that the majority of the 30 patients had moderate non-complicated episodes of diverticulitis that resolved with antibiotic therapy while the study drug was maintained. The patient, who had developed a sigmoid abscess recovered with antibiotic therapy.

The excess of diverticulitis occurrences in patients treated with eplivanserin versus placebo was identified as a safety signal. Specific measures to minimize the identified risk are proposed to minimise risk to individual patients treated with eplivanserin.

**Table 1: Incidence of diverticulitis by sub-group, in eplivanserin-treated population (all eplivanserin doses)**

| **Incidence of Diverticulitis by sub-group** **n/N(%) (95% Cl)** | **All eplivanserin doses** **(N=3030)** |
|---|---|
| Gender | |
| Male | 12/1379 (0.9%) (0.45 to 1.52) |
| Female | 18/1651 (1.1%) (0.65 to 1.72) |
| | |
| Age groups (years) | |
| <65 | 18/2636 (0.7%) (0.41 to 1.08) |
| >=65 | 12/394 (3.0%) (1.58 to 5.26) |
| | |
| Race | |
| Caucasian | 29/2730 (1.1%) (0.71 to 1.52) |
| Black | 1/162 (0.6%) (0.02 to 3.39) |
| Asian/Oriental/Other | 0/138 |
| | |
| BMI (kg/m2) | |
| < 25 | 6/1121 (0.5%) (0.20 to 1.16) |
| 25-30 | 17/1292 (1.3%) (0.77 to 2.10) |
| >=30 | 7/617 (1.1%) (0.46 to 2.32) |
| | |
| Creatinine clearance at baseline (ml/min) | |
| Normal: >=80 or Missing | 18/2317 (0.8%) (0.46 to 1.23) |
| Mild : [50;80[ | 11/669 (1.6%) (0.82 to 2.92) |
| Moderate/Severe : <50 | 1/44 (2.3%) (0.06 to 12.02) |
| | |
| Medical history of diverticulitis | |
| No | 23/3005 (0.8%) (0.49 to 1.15) |
| Yes | 7/25 (28.0%) (12.07 to 49.39) |
| Medical history of diverticular disease* | |
| No | 18/2984 (0.6%) (0.36 to 0.95) |
| Yes | 12/46 (26.1%) (14.27 to 41.13) |
| Medical history of constipation | |
| No | 27/2943 (0.9%) (0.61 to 1.33) |
| Yes | 3/87 (3.4%) (0.72 to 9.75) |
| | |
| Concomitant drugs that inhibit prostaglandin synthesis | |
| No | 15/2161 (0.7%) (0.39 to 1.14) |
| Yes | 15/869 (1.7%) (0.97 to 2.83) |
| Concomitant opioids | |
| No | 27/2999 (0.9%) (0.59 to 1.31) |
| Yes | 3/31 (9.7%) (2.04 to 25.75) |

| | |
|---|---|
| * Diverticular disease includes diverticulitis | |

## Claims

1. Eplivanserin or pharmaceutically acceptable salts or esters thereof for the treatment of sleep disorders in patients suffering from sleep disorders and not displaying a prior history of diverticulitis, a therapeutic amount of eplivanserin or pharmaceutically acceptable salts or esters thereof being administered.

2. Eplivanserin according to claim 1, wherein a pharmaceutically acceptable salt of eplivanserin is hemifumarate.

3. Eplivanserin according to claim 1 or 2, wherein sleep disorders are insomnia.

4. Eplivanserin according to claim 1 or 2, wherein sleep disorders are chronic insomnia.

5. Eplivanserin according to anyone of claims 1 to 4, wherein sleep disorders are insomnia **characterized by** difficulties with sleep maintenance.

6. Eplivanserin according to anyone of claims 1 to 5, wherein sleep disorders are chronic insomnia **characterized by** difficulties with sleep maintenance.

7. Eplivanserin according to anyone of claims 1 to 6, wherein a therapeutic amount of eplivanserin is 5 mg once a day.

8. Eplivanserin according to anyone of claims 1 to 6, wherein the administration can be made at any time of the day.

9. Eplivanserin according to claim 8, wherein the administration can be made in the morning.

10. Eplivanserin according to claim 8, wherein the administration can be made in the evening.

11. Method of providing eplivanserin or pharmaceutically acceptable salts or esters, wherein said eplivanserin or pharmaceutically acceptable salts or esters thereof is provided along with information indicating that eplivanserin or pharmaceutically acceptable salts or esters thereof is useful for treating patients suffering from sleep disorders and not displaying a prior history of diverticulitis.

12. Method according to claim 10, wherein a pharmaceutically acceptable salt of eplivanserin is hemifumarate.

13. Method according to claim 10, wherein sleep disorders are insomnia.

14. Method according to claim 10 or 11, wherein sleep disorders are chronic insomnia.

15. Method according to anyone of claims 10 to 14, wherein sleep disorders are insomnia **characterized by** difficulties with sleep maintenance.

16. Method according to anyone of claims 10 to 15, wherein sleep disorders are chronic insomnia **characterized by** difficulties with sleep maintenance.

17. Method according to claim 11, wherein the information comprises printed matter that advises that eplivanserin or pharmaceutically acceptable salts or esters thereof is useful for treating patients suffering from sleep disorders and not displaying a prior history of diverticulitis.

18. Method according to claim 17 wherein said printed matter is a label.

19. Method of managing the risk of diverticulitis to allow an effective and safe use of eplivanserin or pharmaceutically acceptable salts or esters thereof in the treatment of patients treated for sleep disorders, said method comprising the following steps:
a) Initially check the patients suffering from sleep disorders,
b) if abdominal pain associated with altered gastrointestinal mobility or fever is detected during the treatment with eplivanserin or pharmaceutically acceptable salts or esters thereof, then the treatment should be discontinued;
c) if, after a diagnosis of diverticulitis is excluded, treatment with eplivanserin or pharmaceutically acceptable salts or esters thereof should be reinitiated, patients should be closely monitored.

20. Method according to claim 19, wherein a pharmaceutically acceptable salt of eplivanserin is hemifumarate.

21. Method according to claim 19, wherein sleep disorders are insomnia.

22. Method according to anyone of claims 19 to 21, wherein sleep disorders are chronic insomnia.

23. Method according to anyone of claims 19 to 20, wherein sleep disorders are insomnia **characterized by** difficulties with sleep maintenance.

24. Method according to anyone of claims 19 to 21, wherein sleep disorders are chronic insomnia **characterized by** difficulties with sleep maintenance.

25. Method according to claim 19, which method comprises the following steps:
a) Initially check the patients suffering from sleep disorders,
b) if abdominal pain usually in the lower left quadrant associated with altered gastrointestinal mobility, such as constipation or diarrhoea, or fever, is detected during the treatment with eplivanserin or pharmaceutically acceptable salts or esters thereof, then the treatment with eplivanserin or pharmaceutically acceptable salts or esters thereof should be discontinued;
c) if, after a diagnosis of diverticulitis is excluded, treatment with eplivanserin or pharmaceutically acceptable salts or esters thereof should be reinitiated, patients should be closely monitored.

26. Method according to claim 25, wherein a pharmaceutically acceptable salt of eplivanserin is hemifumarate.

27. Method according to claim 25, wherein sleep disorders are insomnia.

28. Method according to anyone of claims 25 to 27, wherein sleep disorders are insomnia.

29. Method according to anyone of claims 25 to 28, wherein sleep disorders are chronic insomnia.

30. Method according to anyone of claims 25 to 29, wherein sleep disorders are chronic insomnia **characterized by** difficulties with sleep maintenance.

31. A method of promoting the use of eplivanserin or pharmaceutically acceptable salts or esters thereof, the method comprising the step of conveying to a recipient at least one message selected from the group consisting of:
(a) eplivanserin or pharmaceutically acceptable salts or esters thereof should be prescribed to a patient who has not been diagnosed with diverticulitis;
(b) eplivanserin or pharmaceutically acceptable salts or esters thereof should be prescribed to a patient who does not have a prior history of diverticulitis;
(c) eplivanserin or pharmaceutically acceptable salts or esters thereof is contraindicated in patients with a prior history of diverticulitis;
(d) if abdominal pain associated with altered gastrointestinal mobility or fever is detected during the treatment with eplivanserin or pharmaceutically acceptable salts, then the treatment with eplivanserin or pharmaceutically acceptable salts or esters thereof should be discontinued.

32. Method according to claim 31, wherein a pharmaceutically acceptable salt of eplivanserin is hemifumarate.

33. Method of providing eplivanserin or pharmaceutically acceptable salts or esters thereof, wherein said eplivanserin or pharmaceutically acceptable salts or esters thereof is useful for treating patients suffering from sleep disorders and not displaying a prior history of diverticulitis.

34. An article of manufacture comprising
a) a packaging material;
b) eplivanserin or pharmaceutically acceptable salts or esters thereof or, and
c) a label or package insert contained within the packaging material indicating that:
i) eplivanserin or pharmaceutically acceptable salts or esters thereof is contraindicated in patients with a history of diverticulitis and
ii) if abdominal pain associated with altered gastrointestinal mobility or fever is detected during the treatment with eplivanserin or pharmaceutically acceptable salts or esters thereof, then the treatment with eplivanserin or pharmaceutically acceptable salts or esters thereof should be discontinued.

35. An article according to claim 34, wherein a pharmaceutically acceptable salt of eplivanserin is hemifumarate.

36. A package comprising eplivanserin or pharmaceutically acceptable salts or esters thereof and a label, said label comprising a printed statement which informs a prospective user that:
i) eplivanserin or pharmaceutically acceptable salts or esters thereof is contraindicated in patients with a history of diverticulitis and
ii) if abdominal pain associated with altered gastrointestinal mobility or fever is detected during the treatment with eplivanserin or pharmaceutically acceptable salts or esters thereof, then the treatment with eplivanserin or pharmaceutically acceptable salts or esters thereof should be discontinued.

37. A package according to claim 36, wherein a pharmaceutically acceptable salt of eplivanserin is hemifumarate.
